# FASCICULE DE BREVET EUROPEEN

(11) **EP 2 197 913 B1**
(45) Date de publication et mention de la délivrance du brevet: **21.01.2015**
(21) Numéro de dépôt: 08842858.6
(22) Date de dépôt: 10.10.2008
(51) Int. Cl.: C07K 16/16

(54) **ANTICORPS ANTI RICINE**
ANTI-RICIN-ANTIKÖRPER
ANTI-RICIN ANTIBODY

(30) Priorité: 11.10.2007 FR 0707132
(43) Date de publication de la demande: 23.06.2010
(73) Titulaire: Etat Français représenté par le Délégué Général pour l' Armement, 00457 Armées (FR); Technische Universität Braunschweig / Institut Für Biochemie Und Biotechnologie, 38106 Braunschweig (DE)
(72) Inventeur: THULLIER, Philippe, F-38190 Bernin (FR); HUST, Michael, D-30167 Hannover (DE); DÜBEL, Stefan, D-38108 Braunschweig (DE)
(74) Mandataire: Cabinet Plasseraud
(86) Numéro de dépôt international: PCT/FR2008/051850
(87) Numéro de publication internationale: WO 2009/053637

(56) Documents cités:
- WO-A-2005/020891
- US-A- 5 626 844
- YUGANG WANG ET AL: "Novel chimeric anti-ricin antibody C4C13 with neutralizing activity against ricin toxicity" BIOTECHNOLOGY LETTERS, KLUWER ACADEMIC PUBLISHERS, DO, vol. 29, no. 12, 27 juillet 2007 (2007-07-27), pages 1811-1816, XP019548718 ISSN: 1573-6776
- LEMLEY P V ET AL: "Identification and characterization of a monoclonal antibody that neutralizes ricin toxicity in vitro and in vivo." HYBRIDOMA OCT 1994, vol. 13, no. 5, octobre 1994 (1994-10), pages 417-421, XP000943688 ISSN: 0272-457X
- LAFFLY EMMANUELLE ET AL: "Selection of a macaque Fab with framework regions like those in humans, high affinity, and ability to neutralize the protective antigen (PA) of Bacillus anthracis by binding to the segment of PA between residues 686 and 694" ANTIMICROBIAL AGENTS AND CHEMOTHERAPY, AMERICAN SOCIETY FOR MICROBIOLOGY, WASHINGTON, DC, US, vol. 49, no. 8, 1 août 2005 (2005-08-01), pages 3414-3420, XP002444247 ISSN: 0066-4804
- GOLDMAN ELLEN R ET AL: "Facile generation of heat-stable antiviral and antitoxin single domain antibodies from a semisynthetic llama library." ANALYTICAL CHEMISTRY 15 DEC 2006, vol. 78, no. 24, 15 décembre 2006 (2006-12-15), pages 8245-8255, XP002488539 ISSN: 0003-2700
- MCGUINNESS CAROLYN R ET AL: "Characterization of a novel high-affinity monoclonal immunoglobulin G antibody against the ricin B subunit." INFECTION AND IMMUNITY JUN 2006, vol. 74, no. 6, juin 2006 (2006-06), pages 3463-3470, XP002488540 ISSN: 0019-9567

## Description

La présente demande concerne des anticorps anti ricine. En particulier, la présente demande a pour objet un anticorps anti ricine, dirigé contre la chaîne A de la ricine.

La ricine est une toxine inhibitrice de la synthèse protéique, produite par un arbrisseau de la famille des euphorbiacées, le ricin. La ricine est une glycoprotéine très toxique d'un poids moléculaire d'environ 60-65 kDa, qui est formée de deux chaînes polypeptidiques A et B, reliées entre elles par un pont disulfure. La chaîne B est une lectine qui se lie aux glycoprotéines contenant du galactose et aux glycolipides exprimés à la surface cellulaire, facilitant l'entrée de la ricine dans le cytosol. La chaîne A inhibe la synthèse protéique en inactivant de façon irréversible la sous unité ribosomale 28S.

La ricine est toxique par voie orale, parentérale, et pulmonaire. La ricine dispersée sous forme de poudre ou d'aérosol peut entraîner dans un délai variant de quelques minutes à plusieurs heures des signes d'irritation oculaire (sensation de brûlure, larmoiement, conjonctivite plus ou moins sévère) et pharyngée ainsi qu'une irritation respiratoire plus ou moins marquée : toux, dyspnée, oedème pulmonaire pouvant conduire à un syndrome de détresse respiratoire aigu. Chez l'homme, la dose estimée létale de ricine est de 1 à 10 µg/kg.

Un test de diagnostic rapide de l'intoxication par la ricine par voie pulmonaire a récemment été développé (Guglielmo-Viret et al. 2007).

Après une exposition à la ricine, les antidotes suivants peuvent être utilisés : analogues de sucre empêchant la liaison de la ricine à sa cible ou inhibiteurs de la sous-unité catalytique tels que l'azidothymidine.

Une autre stratégie pour le traitement d'une intoxication à la ricine est la vaccination. Par exemple, des anticorps ont été développés dans le but d'interférer avec la liaison de la toxine de la maladie du charbon aux récepteurs de surface cellulaire ou dans le but d'inhiber l'assemblage de la toxine. Cependant, à l'heure actuelle, aucune thérapie spécifique de la ricine n'est disponible. Seul un anticorps chimérique a été développé par Wang et al. (Wang et al., 2007. Biotechnol Lett 29 :1811-1816). Cet anticorps chimérique est dérivé de l'anticorps murin c4C13. Cet anticorps appartient à la première génération d'anticorps recombinants, connue pour induire des effets secondaires (HACA « human anti chimeric antibody ») et une faible tolérance.

Sont également connus les anticorps anti ricine UNIVAX 70 et 138, qui sont des IgG neutralisants spécifiques de la chaîne A de la ricine se liant à l'épitope neutralisant et permettant de prévenir l'effet toxique de la ricine in vitro (US 5626844).

L'immunisation passive avec des anticorps reste cependant une stratégie efficace pour neutraliser la ricine. Le développement de nouveaux anticorps permettant de neutraliser la ricine est ainsi d'un intérêt général pour une prévention et un traitement efficace d'une intoxication à la ricine.

Dans un travail récent, les inventeurs ont immunisé un macaque avec la chaîne A de la ricine pour obtenir des anticorps destinés à traiter l'infection humaine par la ricine. A partir de la moelle osseuse, les inventeurs ont amplifié les gènes codant des fragments d'anticorps spécifiques de la chaîne A de la ricine et les ont clonés pour obtenir une librairie. La librairie a ensuite été criblée pour isoler des fragments d'anticorps de forte affinité et fortement neutralisants.

Les inventeurs ont ainsi pu isoler un fragment scFv de forte affinité et fortement neutralisant, dénommé 43RCA. L'avantage d'un tel anticorps est que ces régions charpentes présentent notamment une forte identité de séquence avec des régions charpentes d'anticorps humains, et avec des régions charpentes codées par des gènes germinaux humains.

La présente demande a donc pour objet de fournir un anticorps anti ricine, dirigé contre la chaîne A de la ricine.

La présente demande a aussi pour objet une composition comprenant ledit anticorps anti ricine ainsi qu'une composition pharmaceutique comprenant ledit anticorps anti ricine et un véhicule pharmaceutiquement acceptable.

La présente demande a également pour objet l'utilisation dudit anticorps modifié pour la préparation d'une composition pharmaceutique destinée au traitement ou à la prévention d'une intoxication par la ricine.

La présente demande concerne également un kit pour la détection de la ricine, comprenant ledit anticorps nanti ricine ainsi qu'une méthode de détection de la ricine.

La présente demande sera mieux comprise à l'aide des définitions suivantes.

Le terme « anticorps » se réfère à une molécule d'immunoglobuline ou un fragment d'une molécule d'immunoglobuline ayant la capacité de se lier spécifiquement à un antigène particulier. Un anticorps selon la présente invention peut être un anticorps entier (anticorps chimérique, anticorps humanisé, notamment) ou un fragments d'anticorps F(ab')₂, Fab, scFv, Fv, anticorps simple chaîne notamment.

Le terme « ricine » se réfère à toute maladie causée, directement ou indirectement, par une intoxication par la ricine. La ricine ne présente aucune sélectivité pour un type spécifique de cellules. Comme tous les types de cellules peuvent être affectés, les symptômes dépendent du lieu d'entrée de la toxine dans le corps humain. Selon la dose, les premiers symptômes apparaissent après une période de latence de 2 à 24h. Après inhalation d'une dose importante de ricine, la victime peut présenter des troubles généraux (fièvre, sudation, nausées) et des difficultés respiratoires, allant jusqu'à tableau d'oedème pulmonaire. Les troubles des échanges gazeux peuvent conduire à la mort. Après une ingestion orale de la ricine, la victime souffre de vomissements et de diarrhées pouvant devenir sanguinolentes, engendrant une forte déshydratation voire un collapsus. En quelques jours, les fonctions spléniques, hépatiques et rénales peuvent être considérablement dégradées. La mort due à une intoxication à la ricine peut survenir dans les 36 à 72h après intoxication, selon la dose de ricine et la voie d'intoxication.

Le terme « isolé » signifie « amplifié in vitro par PCR », « produit de façon recombinante par clonage », « purifié par séparation sur gel ou par clivage », ou encore « synthétisé par exemple par synthèse chimique ».

Le terme « vecteur » se réfère à un acide nucléique dans lequel la séquence d'intérêt peut être insérée par restriction puis ligation pour le transport entre différents environnements génétiques ou pour l'expression dans une cellule hôte. Les vecteurs sont par exemple les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus. Un vecteur de clonage est un vecteur capable de se répliquer dans une cellule hôte et qui est de plus caractérisé par la présence de un ou plusieurs sites de restrictions par les endonucléases. Un vecteur d'expression est un vecteur dans lequel la séquence d'ADN d'intérêt peut être insérée par restriction ou ligation de telle façon qu'elle puisse être répliquée et/ou transcrite en ARN. Les vecteurs peuvent contenir en outre un ou plusieurs marqueurs de sélection ou d'identification des cellules ayant été transformées ou transfectées avec le vecteur.

Le terme « anticorps humanisé » se réfère à des anticorps d'origine animale dans lesquels des composants humains ont été substitués à certains composants originels.

Le terme « prévention d'une maladie » correspond à la prévention de l'apparition de cette maladie chez un sujet, en particulier un humain, chez qui la maladie ne s'est pas encore déclarée.

Le terme « traitement d'une maladie » correspond à l'inhibition de cette maladie, i.e. l'arrêt de son développement, sa régression, ou à la disparition des symptômes et/ou conséquences de la maladie, ou encore à la disparition des causes de la maladie.

Le terme « quantité thérapeutique effective » se réfère à la quantité qui est suffisante pour effectuer le traitement lorsqu'elle est administrée à un sujet qui nécessite un tel traitement. La quantité thérapeutique effective dépend du sujet, du stade de la maladie à traiter et du mode d'administration, et peut être déterminée par des opérations de routine par l'homme du métier.

Comme il est bien connu, seule une partie de l'anticorps, la région variable, est impliquée dans la liaison de l'anticorps à son épitope. Les régions constantes de l'anticorps activent les effecteurs immunitaires, notamment les phagocytes, les cellules tueuses et le complément, ainsi que d'autres récepteurs tels que le récepteur de Brambell impliqué dans le recyclage biologique des anticorps ; ces régions constantes ne sont pas impliquées dans la liaison à l'antigène. Un anticorps dont la région constante (Fc) a été enzymatiquement clivée de façon à en préserver la région charnière est désigné comme un fragment F(ab')2 et conserve les deux sites de liaison à l'antigène.

De même, un anticorps dont la région constante, y compris la région charnière a été enzymatiquement clivée, ou qui a été produit sans cette région, est désigné comme un fragment Fab et conserve un des deux sites de liaison à l'antigène. Les fragments Fab consistent en une chaîne légère qui est liée de façon covalente à une portion de la chaîne lourde appelée Fd.

Un fragment scFv consiste en la région variable de la chaîne lourde et la région variable de la chaîne légère reliées entre elles par un lien peptidique qui permet aux deux régions variables de s'associer pour former un site de liaison à l'antigène.

Dans la région variable, on trouve les régions déterminant la complémentarité (CDRs, *complementary determining regions*), aussi appelées régions hypervariables, qui interagissent directement avec l'antigène. Modifier les CDRs peut donc permettre de modifier l'affinité d'un anticorps. Dans la région variable, on trouve un second type de régions, appelées régions charpentes (FRs, *frameworks),* qui maintiennent la structure tertiaire des CDRs. Ces régions charpentes sont assez spécifiques de l'espèce où a été produit l'anticorps. Ainsi, dans les régions variables de la chaîne lourde et dans la chaîne légère, on trouve quatre régions charpentes (FR1 à 4) séparées respectivement par trois CDR (CDR1 à 3). Ces régions FR et CDR sont arrangées du domaine N-terminal au domaine C-terminal dans l'ordre suivant : FR1, CDR1, FR2, CDR2, FR3, CDR3, FR4. Les régions CDR3 appartenant aux régions variables de la chaîne lourde et de la chaîne sont particulièrement importantes pour la reconnaissance de l'antigène.

Les inventeurs ont pu mettre en évidence un fragment scFv, dont la région variable de la chaîne lourde a pour séquence SEQ ID N° 1 et dont la région variable de la chaîne légère a pour séquence SEQ ID N°2.

Ce fragment scFv présente une forte affinité égale à 5 10⁻¹¹ M et possède un fort potentiel de neutralisation de la ricine.

La présente invention a pour objet un anticorps anti ricine caractérisé en ce que la région variable de la chaîne lourde comprend les régions CDR1, CDR2 et CDR3 ayant respectivement pour séquences SEQ ID N°5, SEQ ID N°7 et SEQ ID N°3, et la région variable de la chaîne légère comprend les régions CDR1, CDR2 et CDR3 ayant respectivement pour séquences SEQ ID N°6, SEQ ID N°8 et SEQ ID N°4.

Selon un mode encore plus préféré de l'invention, l'anticorps anti-ricine est caractérisé en ce que la région variable de la chaîne lourde a la séquence d'acides aminés représentée par SEQ ID N°1 et la région variable de la chaîne légère a la séquence d'acides aminés représentée par SEQ ID N°2.

La présente invention a également pour objet un fragment scFv, comprenant la séquence SEQ ID N°1 et la séquence SEQ ID N°2 liées entre elle par un lien peptidique tel que le lien (G₄S)x3 ou le lien ASTKGPKLEEGEFSEARV (SEQ ID N°10).

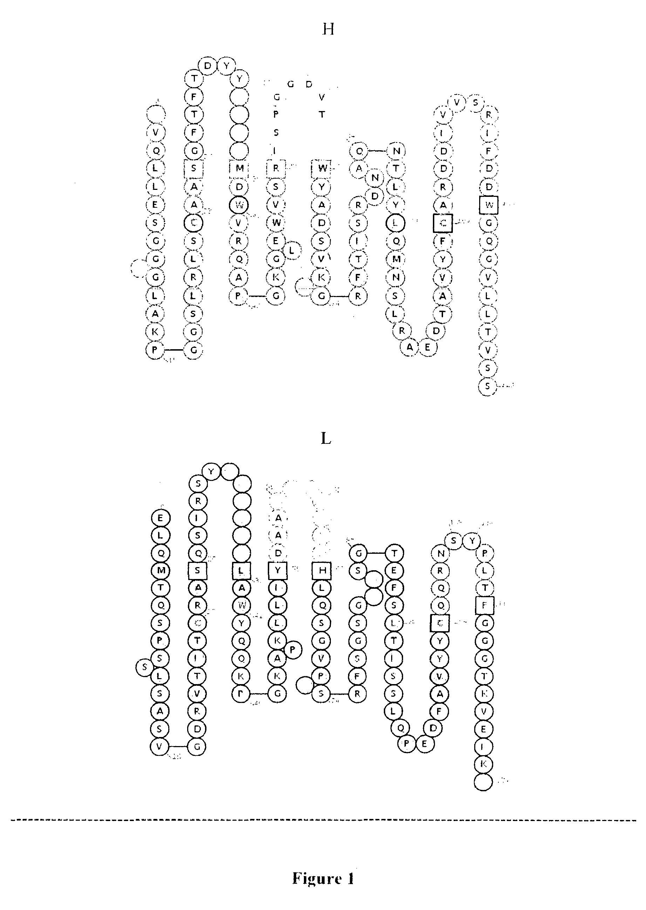

Les séquences SEQ ID N°1 et SEQ ID N°2 sont présentées sous forme de schéma bidimensionnel sur la figure 1. Les séquences d'ADN codant ces séquences SEQ ID N°1 et SEQ ID N°2, correspondent respectivement à FJ 178346 et FJ 178347.

La présente invention a également pour objet un fragment scFv anti-ricine ayant pour séquence la séquence SEQ ID N°9.

L'affinité K_{D} d'un anticorps peut être mesurée par les techniques conventionnelles connues de l'homme du métier. La constante d'affinité de l'anticorps anti ricine de la présente invention a été calculé à partir des constantes d'association et de dissociation mesurées en temps réel par résonance plasmonique de surface tel qu'expliqué dans les exemples.

La présente demande fournit donc des fragment F(ab')2, Fab, Fv, scFv et Fd d'un anticorps anti ricine tel que décrit ci-dessus. De tels fragments incluent aussi des CDRs et des peptides ayant pour séquence une partie de l'anticorps, en particulier de ses CDRs, de ses domaines variables ou une combinaison de ceux-ci.

La présente demande fournit également des anticorps chimériques dans lesquels la partie Fc et/ou les régions constantes du fragment Fd et de la chaîne légère de l'anticorps provient de séquences homologues humaines ou non humaines.

Selon un mode de l'invention, la partie Fc de l'anticorps peut être choisie de façon à produire des IgA, des IgM ou des IgG.

Selon un autre mode de l'invention, la partie Fc de l'anticorps peut être une partie Fc provenant de souris, chevaux, ovins, bovins ou autres mammifères.

La présente demande fournit également des fragments scFv fusionnés à des régions Fc.

Selon un mode préféré de réalisation de la demande, l'anticorps anti ricine selon l'invention possède une partie Fc d'origine humaine. De tels anticorps entiers sont préférés pour l'administration chez l'homme car ils présentent une demi-vie plus longue que des fragments d'anticorps tels que les Fab, et sont plus adaptés à une administration intraveineuse, intra-péritonéale, intramusculaire, sous-cutanée, transdermale ou par aérosol.

Dans certains modes de réalisation de la demande, les fragments Fab ou scFv sont préférés pour les raisons suivantes : a) comme les fragments Fab ou scFv ne possèdent qu'un seul site de liaison à l'antigène, les complexes immuns de grande taille ne peuvent pas se former, b) l'absence de région Fc empêche l'apparition d'une réaction inflammatoire activée par le Fc, telle que l'activation de la cascade du complément, c) la pénétration dans les tissus d'une petite molécule Fab ou scFv est plus facile, et d) la production de Fab ou scFv est facilement réalisée et à faible coût dans des bactéries telles que E. coli.

Ainsi, un objet de la présente demande est de fournir des Fabs de l'anticorps anti ricine selon l'invention, des fragments de cet anticorps plus petits ou plus grands que des fragments Fabs ou encore des peptides de liaison à l'épitope, et en particulier des peptides dérivés des régions hypervariables de l'anticorps anti ricine selon l'invention.

Un autre objet de l'invention est de fournir des variants de l'anticorps anti ricine de la présente invention.

Par variant de l'anticorps anti-ricine de la présente invention, on entend tout variant ayant conservé la capacité de liaison à la ricine A. La détermination de cette capacité de liaison à la ricine A peut être déterminée par des techniques conventionnelles bien connues de l'homme du métier, comme par exemple un test ELISA ou un Western-blot.

Des variants de la séquence d'acides aminés de l'anticorps de la présente invention peuvent être préparés par utilisation d'une partie seulement des séquences nucléotidiques ou peptidiques présentées dans le présent texte. De tels variants comprennent par exemple des délétions et/ou insertions et/ou substitutions de résidus dans la séquence d'acides aminés de l'anticorps. La présente invention concerne tout variant obtenu à partir des séquences présentées dans ce texte dès lors que l'expression de la construction finale possède les caractéristiques attendues d'un anticorps anti ricine. Les changements d'acides aminés peuvent aussi altérer le processus post-traductionnel de l'anticorps, comme par exemple le nombre ou la position des sites de glycosylation.

Les séquences nucléiques codant les séquences d'acides aminés des variants de l'anticorps de la présente invention peuvent être préparées par les méthodes conventionnelles, par exemple la synthèse de gènes, ou la mutagénèse dirigée ou la mutagénèse au hasard suivie d'une étape de sélection des produits obtenus.

Pour analyser les conséquences d'une mutation à un site donné, les variants doivent être testés pour l'activité désirée. En général, la séquence d'acides aminés des variants de l'anticorps de la présente invention a au moins 70% d'identité de séquence avec la séquence originale de la région variable de l'anticorps anti ricine, de préférence au moins 75%, 80%, 85%, 90% et plus préférablement 95% d'identité de séquence avec la séquence originale de la région variable de l'anticorps anti ricine.

L'identité de séquence est définie ici comme le pourcentage d'acides aminés dans la séquence du variant qui sont identiques à ceux de la séquence originale, après avoir aligné les séquences et introduits des espaces, si nécessaire, pour obtenir le maximum d'identité de séquence, et sans considérer les substitutions conservatives (tableau 1) comme faisant parties de l'identité de séquence.

L'identité de séquence peut être déterminée par les méthodes standards en utilisant des programmes tels que BLAST ou FASTA.

Des exemples de variants sont les suivants :
- les variants obtenus par insertion

L'insertion d'acides aminés comprend les fusions N ou C-terminal allant de un résidu à des polypeptides comprenant plus de 100 résidus, ainsi que des insertions à l'intérieur de la séquence de un ou plusieurs acides aminés. Des exemples d'insertions terminales sont un anticorps avec un résidu N-terminal méthionine ou un anticorps fusionné avec un épitope marqueur (HA, c-myc, séquence poly-histidine, glycoprotéine D de HSV) ou encore un anticorps fusionné à un polypeptide qui augmente la demi-vie de l'anticorps.
- les variants obtenus par substitutions

Ces variants sont par exemple obtenus par mutagénèse substitutionnelle dans les régions hypervariables ou charpentes de l'anticorps. Les substitutions conservatives sont montrées dans le tableau 1.

| Acide aminé original | Substitutions possibles | Substitutions préférées |
|---|---|---|
| Ala (A) | val ; leu ; ile | val |
| Arg(R) | lys ; gln ; asn | lys |
| Asn (N) | gln ; his ; asp ; lys ; gln | arg |
| Asp (D) | glu ; asn | glu |
| Cys (C) | ser ; ala | ser |
| Gln (Q) | asn ; ala | ser |
| Glu (E) | asp ; gln | asp |
| Gly (G) | ala | |
| His (H) | asn ; gln ; lys ; arg | |
| Ile (I) | leu ; val ; met ; ala ; phe | leu norleucine |
| Leu (L) | norleucine ; ile ; val met ; ala ; phe | ile |
| Lys (K) | arg ; gln ; asn | arg |
| Met (M) | leu ; phe ; ile | leu |
| Phe (F) | leu ; val ; ile ; ala ; tyr | |
| Pro (P) | ala | |
| Ser (S) | thr | |
| Thr (T) | ser | ser |
| Trp (W) | tyr ; phe | tyr |
| Tyr (Y) | trp ; phe ; the ; ser | phe |
| Val (V) | ile ; leu ; met ; phe ; ala ; norleucine | leu |

Des modifications substantielles dans les propriétés biologiques de l'anticorps de la présente invention peuvent être obtenues par sélection de substitutions qui diffèrent significativement dans leur effet sur le maintien (a) de la structure du squelette polypeptidique au niveau de la substitution, (b) de la charge ou de l'hydrophobicité de la molécule au site cible, ou (c) le volume de la chaîne latérale.

Les résidus naturels peuvent être divisés en différents groupes selon leurs propriétés :
(1) résidus hydrophobes: norleucine, met, ala, val, leu, ile;
(2) résidus neutres hydrophiles: cys, ser, thr;
(3) résidus acides: asp, glu;
(4) résidus basiques: asn, gln, his, lys, arg;
(5) résidus influençant l'orientation de la chaîne: gly, pro; et
(6) résidus aromatiques: trp, tyr, phe.

Les substitutions conservatives impliquent le remplacement d'un acide aminé avec un autre membre du même groupe, tandis que les substitutions non conservatives impliquent le remplacement d'un des groupes par un membre d'un autre groupe.

N'importe quel résidu cystéine non impliqué dans le maintien de la conformation de l'anticorps peut aussi être substitué, en général par une sérine, pour augmenter la stabilité oxydative de la molécule. De même, des liaisons cystéines peuvent être ajoutées pour augmenter la stabilité de l'anticorps (en particulier dans le cas de fragment Fv).
- les variants obtenus par maturation d'affinité

La maturation d'affinité implique la préparation et le criblage de variants d'anticorps qui possèdent des mutations (délétions, insertions, ou substitutions) dans les régions variables de l'anticorps parental, ainsi que la sélection des variants ayant des propriétés biologiques améliorées telle qu'une meilleure affinité de liaison par rapport à l'anticorps parental ou une meilleure tolérance lors de l'administration à l'homme. Une méthode conventionnelle de génération de tels variants d'affinité améliorée est la maturation d'affinité utilisant les techniques de mutagenèse suivies de sélection, par exemple par « phage display ».

Un objet de la présente demande est également de fournir un anticorps anti ricine selon l'invention présentant une meilleure tolérance par le système immunitaire humain. Un tel anticorps humanisé présente l'avantage de ne pas ou de moins induire de réponse immune contre lui-même, et d'avoir une plus longue demi-vie.

La demande a pour objet un anticorps anti-ricine selon l'invention humanisé. Cet anticorps anti-ricine peut être humanisé ou super-humanisé par toute technique connue de l'homme du métier. Les techniques d'humanisation, qui visent à augmenter l'homologie des régions charpentes de l'anticorps avec des régions charpentes codées par des gènes somatiques humains codant des anticorps, sont par exemple décrites dans la revue de Almagro et al. « humanization of antibodies » (Almagro et a., 2008, Front. Biosci. Jan 1;13:1619-33). Des techniques dérivées de l'humanisation, et appelées super-humanisation, ont aussi été décrites : elles peuvent aussi viser à augmenter l'homologie des régions charpentes de l'anticorps avec des régions charpentes codées par des gènes germinaux humains codant des anticorps. Ces techniques ont en commun de parvenir à préserver l'affinité de l'anticorps pour son antigène, alors que les séquences des régions charpentes de cet anticorps ont été modifiées pour en augmenter le degré de similarité avec leurs homologues humains.

Dans un mode de réalisation, un anticorps anti ricine humanisé comprend en outre au moins une mutation dans la région variable de la chaîne lourde sélectionnée dans le groupe constitué par: aucun/Q (1), L/V (5), A/V (13), D/S (40), V/I (42), R/Y (55), W/Y (66), Q/K (84), T/S (86), F/Y (103), V/T (122), L/V (124). La mutation aucun/Q (1) signifie qu'un acide aminé Q (glutamine) est ajouté en position 1. La mutation L/V (5) signifie que l'acide aminé V (valine) remplace l'acide aminé L (leucine) à la position 5. Le positionnement des acides aminés est présenté à la figure 1.

Lorsque le positionnement des acides aminés est calculé à partir de SEQ ID NO : 1, l'anticorps anti ricine selon l'invention est humanisé en comprenant en outre au moins une mutation dans la région variable de la chaîne lourde choisie parmi aucun/Q (1), L/V (5), A/V (12), D/S (35), V/I (37), Y/R (50), W/Y (59), Q/K (76), T/S (78), F/Y (95), V/T (114), L/V (116).

Dans un autre mode de réalisation, l'anticorps anti ricine humanisé comprend en outre au moins une mutation dans la région variable de la chaîne légère sélectionnée dans le groupe constitué par: E/D (1). L/I (2), M/L (4), S/F (10), H/T (66), S/T (88), V/T (101).

Le positionnement des acides aminés est présenté à la figure 1.

Lorsque le positionnement des acides aminés est calculé à partir de SEQ ID NO : 2, l'anticorps anti ricine selon l'invention est humanisé en comprenant en outre au moins une mutation dans la région variable de la chaîne légère choisie parmi E/D (1), L/I (2), M/L (4), S/F (10), H/T (53), S/T (72), V/T (85).

Dans un autre mode de réalisation, l'anticorps anti ricine selon l'invention humanisé, comprend en outre au moins une mutation dans la région variable de la chaîne légère sélectionnée dans le groupe constitué par: E/D(1), L/I(2), S/T (10), H/S(66), Q/E(68), S/T(88), E/D(97), V/T(101). Le positionnement des acides aminés est présenté à la figure 1.

Lorsque le positionnement des acides aminés est calculé à partir de SEQ ID NO : 2, l'anticorps anti ricine selon l'invention est humanisé en comprenant en outre au moins une mutation dans la région variable de la chaîne légère choisie parmi : E/D(1), L/I(2), S/T(10), H/S(53), Q/E(55), S/T(72), E/D(81), V/T(85).

Dans un mode de réalisation préféré l'anticorps anti ricine humanisé comprend en outre au moins une mutation dans la région variable de la chaîne lourde telle que décrite ci-dessus et au moins une mutation dans la région variable de la chaîne légère telle que décrite ci-dessus.

D'après la description ci-dessus des séquences d'acides aminés de la région variable de la chaîne lourde et de la région variable de la chaîne légère des anticorps anti ricine selon l'invention, l'homme du métier est capable de synthétiser, ou de faire synthétiser, des acides nucléiques qui codent ces séquences d'acides aminés.

La présente invention a donc pour objet un acide nucléique codant un anticorps anti ricine selon l'invention.

La présente invention a également pour objet un vecteur comprenant ledit acide nucléique.

Ces acides nucléiques pourront être compris dans un vecteur recombinant pour le clonage ou pour l'expression des anticorps de l'invention.

La présente demande inclut tous les vecteurs recombinants contenant des séquences codantes pour la transformation, transfection ou thérapie génique eucaryote ou procaryote. De tels vecteurs pourront être préparés selon les techniques conventionnelles de biologie moléculaire et comprendront en outre un promoteur approprié, optionnellement une séquence signal pour l'export ou la sécrétion, et des séquences régulatrices nécessaires pour la transcription de la séquence nucléotidique. Un polypeptide de fusion peut être utile pour la purification des anticorps de la présente invention. Le domaine de fusion peut par exemple inclure une queue poly-histidine qui permet la purification sur des colonnes Ni+, ou une ancre membranaire de phage filamenteux qui est particulièrement utile pour le screening de banque, selon la technologie du « phage display ».

Un des vecteurs approprié dans le cadre de l'invention est une molécule d'ADN recombinante adaptée pour recevoir et exprimer une première et une seconde séquence d'ADN, de façon à permettre l'expression d'anticorps hétérodimérique tel qu'un anticorps de longueur complète ou des fragments F(ab')2 ou Fab selon l'invention. Un tel vecteur fournit un système pour indépendamment cloner les deux séquences d'ADN dans deux cassettes séparées présentes dans le vecteur, de façon à former deux cistrons séparés pour l'expression d'un premier et d'un second polypeptide de l'anticorps hétérodimérique. Un tel vecteur d'expression est appelé vecteur di-cistronique.

Les anticorps modifiés de la présente invention peuvent être produits dans des cellules eucaryotes telles que des CHO ou des hybridomes humain ou murin par exemple, ainsi que dans des cellules végétales.

La présente demande a également pour objet des cellules hôtes, procaryotes ou eucaryotes, comprenant un vecteur selon l'invention.

Un autre objet de la présente invention est de fournir une composition comprenant au moins un anticorps anti ricine selon l'invention.

La présente invention a également pour objet une composition pharmaceutique comprenant au moins un anticorps anti ricine selon l'invention et au moins un véhicule pharmaceutiquement acceptable. Un tel véhicule correspond au sens de l'invention à un matériel non toxique qui n'interfère pas avec l'efficacité de l'activité biologique, des ingrédients actifs de la composition. Le terme « pharmaceutiquement acceptable » préfère à un matériel non toxique qui est compatible avec un système biologique tel qu'une cellule, une culture cellulaire, un tissu ou un organisme. Les caractéristiques du véhicule dépendront du mode d'administration.

La présente invention a également pour objet un vaccin ou un médicament comprenant au moins un anticorps anti ricine selon l'invention.

La présente invention concerne l'utilisation d'au moins un anticorps anti ricine selon l'invention pour la préparation d'une composition pharmaceutique, d'un médicament ou d'un vaccin destiné au traitement ou à la prévention d'une intoxication par la ricine.

L'anticorps anti ricine selon l'invention peut être marqué. Des exemples de marqueurs comprennent les enzymes, les radio-isotopes, les composés fluorescents, les métaux colloïdes, les composés chimioluminescents, et les composés bioluminescents. Les méthodes de liaison d'un marqueur à un anticorps sont bien connues de l'homme du métier.

Une autre technique de marquage consiste à coupler l'anticorps à des haptènes de faibles poids moléculaire, ces haptènes pouvant être spécifiquement modifiés au moyen d'une seconde réaction. Des exemples d'haptènes sont la biotine, qui réagit avec l'avidine: ou le dinitrophenol, le pyridoxal ou la fluorescéine, qui peuvent réagir avec des anticorps spécifiques anti-haptènes.

Un objet de la présente demande est de fournir un kit pour la détection de la chaîne A de la ricine. Ce kit comprend :
- un container comprenant au moins un anticorps anti ricine selon l'invention et qui peut être ou non marqué.
- optionnellement, un container comprenant des solutions tampons
- et optionnellement un container comprenant des moyens de détection dudit anticorps anti ricine marqué, tel qu'une protéine de liaison à la biotine, par exemple l'avidine ou la streptavidine, liée à une molécule rapporteur, telle qu'un marqueur fluorescent ou enzymatique. Ce container peut aussi comprendre des moyens de détection dudit anticorps anti ricine non marqué, soit essentiellement des anticorps ou fragments d'anticorps.

L'anticorps anti ricine de la demande peut être utilisé *in vitro,* par exemple dans des tests immunologiques dans lesquels ils sont utilisés en phase liquide ou liés à un véhicule de phase solide. Des exemples de véhicules bien connus sont le verre, le polystyrène, le polypropylène, le polyéthylène, le dextran, le nylon, l'amylase, la cellulose naturelle ou modifiée, le polyacrylamide, l'agarose ou la magnétite. Des exemples de tests immunologiques utilisant l'anticorps anti PA de l'invention sont des radioimmunoessais, des marquages histoimmunologiques, des ELISA, des western-blots, des essais d'immuno-précipitation, des essais d'immuno-diffusion, des essais de fixation du complément, des analyses au FACS ou encore des analyses par puces à protéines.

La présente invention a pour objet de fournir une méthode de détection *in vitro* de la chaîne A de la ricine, dans un échantillon biologique, comprenant :
- la mise en contact de l'échantillon avec au moins un anticorps anti ricine selon l'invention, et
- la détection dudit anticorps anti ricine comme indicateur de la présence de ladite ricine.

L'échantillon biologique peut être liquide : par exemple de la salive, de l'urine, du fluide cérébrospinal, du sérum ou du sang, ou solide ou semi-solide, par exemple des tissus ou des matières fécales ou un tissu solide tel qu'utilisé couramment en diagnostic histologique.

La présente demande a également pour objet de fournir une méthode de détection *in vivo* de la chaîne A de la ricine, dans laquelle un anticorps anti ricine selon la présente invention marqué est administré a un sujet. La quantité d'anticorps marqué administrée doit être suffisante pour permettre la détection de la liaison de l'anticorps à la toxine. La quantité d'anticorps marqué administrée dépendra des facteurs tels que l'âge et le sexe du sujet, ainsi que du stade de la maladie. La quantité administrée peut varier entre 0.01 mg/kg et 50 mg/kg, préférablement entre 0.1 mg/kg et 20 mg/kg, et plus préférablement entre 0.1 mg/kg et 2 mg/kg.

Pour effectuer le diagnostic in vivo, l'anticorps anti ricine de la demande doit être lié à un radioisotope directement ou indirectement par l'intermédiaire de groupes fonctionnels. Des groupes fonctionnels couramment utilisés sont par exemple l'acide diéthylène-triamine-pentacétique (DTPA) et l'acide éthylène-diamine-tétraacétique (EDTA). Des exemples d'ions métalliques radioisotopes sont ¹¹¹In, ⁹⁷Ru, ⁶⁷Ga, ⁶⁸O ⁷²As, ⁸⁹Zr et ²⁰¹Tl.

Les anticorps anti ricine de la demande peuvent aussi être marqués avec un isotope paramagnétique pour le diagnostic par imagerie de résonance magnétique (IRM) ou par résonance de spin électronique (ESR). Des radioisotopes gamma émetteurs de positrons peuvent également être utilisés, tels que ¹⁵⁷Gd, ⁵⁵Mn, ¹⁶²Dy, ⁶⁸Ga, ⁵²Cr, et ⁵⁶Fe.

Les anticorps anti ricine de la demande peuvent également être utilisés *in vitro* ou *in vivo* pour suivre l'évolution du traitement de la maladie, par exemple en déterminant l'augmentation ou la diminution du nombre de cellules ciblées par la ricine ou les changements dans la concentration de ricine dans un échantillon biologique.

La présente demande a pour objet une méthode de traitement d'un sujet, de préférence un humain, susceptible d'être intoxiqué par la ricine, dans laquelle une quantité thérapeutiquement efficace d'un anticorps anti ricine selon l'invention est administré audit sujet.

Une quantité thérapeutiquement efficace correspond à une quantité suffisante pour diminuer les symptômes de la maladie et l'évolution de l'infection. Cette quantité peut varier avec l'âge, le sexe du sujet et le stade de la maladie et sera déterminée par l'homme du métier. Une quantité thérapeutiquement efficace peut varier entre 0.01 mg/kg et 50 mg/kg, préférablement entre 0.1 mg/kg et 20 mg/kg, et plus préférablement entre 0.1 mg/kg et 2 mg/kg, en une ou plusieurs administrations quotidiennes, pendant un ou plusieurs jours.

Le mode d'administration peut être par injection ou par perfusion graduelle. L'injection peut être intraveineuse, intra-péritonéale, intramusculaire, sous-cutanée ou transdermale. Un autre mode d'administration peut être par aérosol.

Les préparations pour une administration parentérale peuvent inclure des solutions aqueuses ou non-aqueuses stériles, des suspensions ou des émulsions. Des exemples de solvents non-aqueux sont le propylène glycol, le polyéthylène glycol, des huiles végétales, telle que l'huile d'olive, ou des esters organiques injectables tels que l'éthyl oléate. Des véhicules aqueux comprennent l'eau, des solutions alcool/eau, des émulsions ou des suspensions.

La présente invention a également pour objet un immunoconjugué comprenant un anticorps anti ricine selon l'invention lié, de façon directe ou indirecte, à un agent thérapeutique.

De tels agents thérapeutiques comprennent des agents chimiques, des radionucléides, des agents immunothérapeutiques, des cyrtokines, des chimiokines, des toxines ou des inhibiteurs d'enzyme. Des exemples de toxines sont la chaîne A de la diphtérie, la chaîne A de l'exotoxine, la chaîne A de l'abrine, la chaîne A de la modeccin, l'alpha-sarcin, les protéines Aleurites fordii, les protéines dianthines, les protéines Phytolaca americana, l'inhibiteur momordica charantia, la curcine, la crotine, l'inhibiteur sapaonaria officinalis, la gélonine, la mitogélline, la restrictocine, la phénomycine, l'énomycine et le tricothecenes. Des exemples de radionucléide sont ²¹²Bi, ¹³¹I, ¹³¹In, ⁹⁰Y, et ¹⁸⁶Re.

La présente demande sera mieux comprise à l'aide du complément de description qui va suivre, qui se réfère à un exemple d'obtention de l'anticorps anti ricine selon l'invention.

Dans les exemples qui suivent, donnés à titre illustratif, il sera fait référence aux figures en annexe:
Figure 1 : schéma en collier de perles de la région variable de la chaîne lourde et de la région variable de la chaîne légère de l'anticorps anti ricine.

La représentation IMGT en collier de perles est réalisée en accord avec la numérotation IMGT.

### Matériels et méthodes

### Souches E. coli

Les souches E. coli suivantes ont été utilisées :
- XL1 (Stratagène, La jolla, CA) : recA1, endA1, gyrA96 thi-1 hsdR17 sup E44 rclA1 lac [F'proAB lacIqZΔM15 Tn10(Tetr)]
- SURE (Stratagène): e14(McrA) Δ(mcrCB-hsdSMR-mrr)171 endA1 supE44 thi-1 gyrA96 relA1 lac recB recJ sbcC umuC::Tn5 (Kanr) uvrC [F' proAB lacIqZΔM15 Tn10 (Tetr)]
- HB2151, utilisées pour l'expression de scFv solubles.

### Toxines

La chaîne A de la ricine, injectée au macaque, a été achetée chez Vector Labs, de même que la ricine complète.

### Construction de la bibliothèque d'anticorps anti ricine

Un macaque (*Macaca fascicularis*) a été anesthésié (0.8 ml d'Imalgene®, Marial, Lyon, France) et 5 ml de moelle osseuse ont été prélevés avant la première et la dernière injection (Jour 0) de chaîne A de la ricine, réalisées afin d'immuniser l'animal. En outre, des prélèvements ont eu lieu régulièrement après J0 et pendant un mois.

L'ARN de ces échantillons de moelle osseuse a été isolé à l'aide de Trizol® (Euromedex, France) et l'ADNc simple brin a été généré à l'aide du système de Préamplification Superscript (Invitrogen, Cergy Pantoise, France) en présence d'oligodT aléatoires.

L'amplification par réaction de la polymérase en chaîne (PCR) a été réalisée dans les mêmes conditions que décrites dans Glamann and Hirsch, 2000 avec les neuf combinaisons d'amorces IgH et les sept combinaisons d'amorces IgK, de façon à générer les produits de PCR H et L respectivement.

Les produits de PCR H et L ont tout d'abord été insérés dans le vecteur pGemT (Promega, Madison, Wiscontin) pour construire une première sous-bibliothèque d'ADN codant la chaîne lourde (fragments Fd) et une seconde sous-bibliothèque d'ADN codant la chaîne légère. L'ADNc dans pGEM a été réamplifié avec des sets de deux amorces oligonucléotidiques pour introduire des sites de restrictions. Un set d'amorce oligonucléotidique kappa humaine et macaque a été utilisée en tant qu'amorces oligonucléotidiques sens, et seul un set spécifique du macaque a été utilisé en tant qu'amorces oligonucléotidiques anti-sens (table I).

Chaque PCR a été réalisée en utilisant la Taq Red (Sigma, Hamburg, Germany) pendant 20 cycles (30 s 94°C, 30 s 57°C, 30 s 72°C). Les produits de PCR ont été séparés par électrophorèse sur gel d'agarose puis purifiés. Les produits de PCR amplifiés VL ou VH ont été regroupés. La bibliothèque a été construite en deux étapes :les fragments VL ont été insérés dans pHAL14, puis les fragments VH ont été insérés dans pHAL14 contenant le répertoire VL. Le vecteur pHAL14 est dérivé du vecteur phagemide pHAL1.

pHAL14 et les fragments VL ont été digérés avec MluI et Notl (NEB, Frankfurt, Germany), les enzymes ont été inactivées, pHAL14 a été déphosphorylé en utilisant de la phosphatase intestinale de veau (MBI Fermentas) et l'ADN a été purifié. Les produits de PCR VL ont été insérés dans la préparation pHAL14 déphosphorylée. L'ADN a été précipité à partir des mélanges de réaction avec de l'éthanol et de l'acétate de sodium et le culot a été lavé deux fois dans de l'éthanol 70%. Les plasmides (bibliothèque de la chaîne VL) ont été isolés à l'aide du kit Plasmid Midi (Qiagen, Hilden, Germany). La bibliothèque de chaîne VL et les fragments VH ont été digérés avec NcoI et IndIII (NEB), et la liaison et l'électroporation ont ensuite été réalisée tel que décrit pour VL.

### Sélection d'anticorps par phage

Les particules de phages-scFv ont été purifiés et concentrées à partir de 50 ml de culture par précipitation au PEG, puis re-suspendues dans 3 ml de PBS-BSA 1%-azide 0,02% et filtrées sur un filtre de 0,45 µm. Le titre de cette préparation de phage était d'environ 5 10¹⁰ pfu/ml. Les phages-scFv ont été soumis à trois cycles d'infection-sélection-récupération tel que précédemment décrit (Andris-Widhopf. Rader et al. 2000).

### Expression de scFv soluble, extraction périplasmique et purification

Chaque variant ADN a été transformé dans des bactéries de la souche d'*E. coli* appelée HB2151, rendues chimiquement compétentes. Les cellules ont été cultivées à 30°C, agitées à 250 rpm dans 1L de milieu SB contenant 50 µg/ml de carbénicilline et 0.1% de glucose. Lorsque la culture a atteint une A₆₀₀ de 1,5, une induction avec 1 mM d'IPTG a été effectuée pendant 18h à 22°C.

Les scFvs ont été extraits avec du sulfate de polymixine B (Sigma) et purifiés sur colonne de nickel (Ni-NTA spin column, QIAGEN, Valencia, CA) selon les instructions du fabricant, puis dialysés avec du PBS 1X à 4°C pendant 3h.

### Quantification du scFv

La pureté du Fab a été testée par SDS-PAGE et sa concentration a été déterminée à l'aide du logiciel Quantity One® (Biorad).

### Mesure en temps réel de la résonance plasmonique de surface (SPR)

Les constantes de cinétique de l'interaction entre la ricine et les scFv obtenus précédemment ont été déterminées en utilisant le système Biacore X SPR (BIAcore, Uppsala, Sweden). La ricine a été immobilisée sur une puce sensible CM5 en utilisant une procédure de couplage des amines par injection de 30 µl de 2 µg/ml de ricine dans 1.0 mM de sodium acétate pH 4,5. Pour minimiser la probabilité de reliaison, le K_{D} a été mesuré en utilisant un débit élevé (30 µl/min) et une quantité minimale d'antigène couplé (environ 500 RU, unités de résonance). Le taux de liaison de différentes concentrations de scFv allant de 5 à 400 nM dans du PBS a été déterminé à un débit de 30 µl/min. Les données de liaison ont été introduites dans un modèle langmuir 1:1 du logicien d'évaluation BIA. Les constantes d'association et de dissociation (kₒₙ et k_{off} respectivement) pour la liaison du scFv à la ricine ont été déterminées à 35°C.

### Analyse de séquences

Les séquences des régions variables des chaînés lourde et légère des clones sélectionnés ont été déterminées par Genome Express (Meylan, France) en utilisant les amorces Mkmyc and MkpelB, (Kirsch et al., 2005). Les séquences ont été analysées en ligne, en utilisant le système IMGT (hltp:/imgt.cines.tr).

### Résultats

Les inventeurs ont ainsi mis en évidence un fragment scFv, anti ricine, le fragment 43RCA selon l'invention, dont l'affinité mesurée par résonance plasmonique de surface est égale à 5 10⁻¹¹ M.

Un test de neutralisation de la toxicité de la ricine a ensuite été mis en place dans le laboratoire.

Des cellules J774.2, de type macrophage, ont été misses en culture à raison de 14000 cellules par puits de 200 µl. L'incubation de ces cellules pendant 24h avec 15 ng/ml de ricine, conduit à la mort de 100% des cellules.

La co-incubation de la ricine avec les différents scFV issus de la librairie a permis d'étudier la capacité de neutralisation de ces fragments d'anticorps.

Les résultats de ce test de neutralisation montre que le fragment d'anticorps 43RCA selon l'invention neutralise 50% de la toxicité à une concentration de 30 ng/ml (CI₅₀ = 30 ng/ml).

## Revendications

1. Anticorps anti ricine, **caractérisé en ce que** la région variable de la chaîne lourde a la séquence d'acides aminés représentée par SEQ ID N°1 et la région variable de la chaîne légère a la séquence d'acides aminés représentée par SEQ ID N°2.

2. ScFv anti ricine ayant pour séquence la séquence SEQ ID N°9.

3. Anticorps anti-ricine selon l'une quelconque des revendications **1** à **2,** ledit anticorps étant en outre humanisé ou super-humanisé.

4. Anticorps anti-ricine selon la revendication **3,** ledit anticorps comprenant au moins une mutation dans la région variable de la chaîne lourde choisie parmi aucun/Q (1), LV (5), A/V (12), D/S (35), V/I (37), Y/R (50), W/Y (59), Q/K (76), T/S (78), F/Y (95), V/T (114) et L/V (116), le positionnement des acides aminés étant calculé à partir de SEQ ID NO :1.

5. Anticorps anti-ricine selon la revendication **3 ou 4,** ledit anticorps comprenant au moins une mutation dans la région variable de la chaîne légère choisie parmi E/D (1), L/I (2), M/L (4), S/F (10), H/T (53), S/T (72) et V/T (85), le positionnement des acides aminés étant calculé à partir de SEQ ID NO :2, ou au moins une mutation dans la région variable de la chaîne légère choisie parmi E/D(1), L/I(2), S/T(10), H/S(53), Q/E(55), S/T(72), E/D(81) et V/T(85), le positionnement des acides aminés étant calculé à partir de SEQ ID NO :2.

6. Acide nucléique codant un anticorps selon l'une quelconque des revendications **1** à **5.**

7. Vecteur comprenant un acide nucléique selon la revendication **6.**

8. Cellule hôte comprenant un vecteur selon la revendication **7.**

9. Composition comprenant au moins un anticorps selon l'une quelconque des revendications **1** à **5.**

10. Composition pharmaceutique comprenant au moins un anticorps selon l'une quelconque des revendications **1** à **5** et au moins un véhicule pharmaceutiquement acceptable.

11. Vaccin comprenant au moins un anticorps selon l'une quelconque des revendications **1** à **5.**

12. Méthode pour la détection *in vitro* de la chaîne A de la ricine dans un échantillon biologique, comprenant :
- la mise en contact de l'échantillon avec au moins un anticorps selon l'une quelconque des revendications **1** à **5,** et
- la détection de la liaison dudit anticorps comme indicateur de la présence de ladite chaîne A de la ricine.

13. Immunoconjugué comprenant un anticorps selon l'une quelconque des revendications **1** à **5** lié à un agent thérapeutique.

## Patentansprüche

1. Anti-Rizin-Antikörper, **dadurch gekennzeichnet, dass** die variable Region der schweren Kette die Aminosäuresequenz SEQ ID Nr: 1 und die variable Region der leichten Kette die Aminosäuresequenz SEQ ID Nr: 2 umfassen.

2. ScFv anti Rizin, dessen Sequenz die Sequenz SEQ ID Nr: 9 aufweist.

3. Anti-Rizin-Antikörper nach einem der Ansprüche 1 bis 2, wobei der Antikörper zudem humanisiert oder super-humanisiert ist.

4. Anti-Rizin-Antikörper nach Anspruch 3, wobei der Antikörper mindestens eine Mutation in der variablen Region der schweren Kette ausgewählt aus keiner/Q (1), L/V (5), A/V (12), D/S (35), V/I (37), Y/R (50), W/Y (59), Q/K (76), T/S (78), F/Y (95), V/T (114) und L/V (116) umfasst, wobei die Positionierung der Aminosäure ab der SEQ ID Nr: 1 berechnet wird.

5. Anti-Rizin-Antikörper nach Anspruch 3 oder 4, wobei der Antikörper mindestens eine Mutation in der variablen Region der leichten Kette ausgewählt aus E/D (1), L/I (2), M/L (4), S/F (10), H/T (53), S/T (72) und V/T (85) umfasst, wobei die Positionierung der Aminosäure ab der SEQ ID Nr: 2 berechnet wird, oder mindestens eine Mutation in der variablen Region der leichten Kette ausgewählt aus E/D (1), L/I (2), S/T (10), H/S (53), Q/E (55), S/T (72), E/D (81) und V/T (85) umfasst, wobei die Positionierung der Aminosäure ab der SEQ ID Nr: 2 berechnet wird.

6. Nukleinsäure kodierend einen Antikörper nach einem der Ansprüche 1 bis 5.

7. Vektor umfassend eine Nukleinsäure nach Anspruch 6.

8. Wirtszelle umfassend einen Vektor nach Anspruch 7.

9. Zusammensetzung umfassend mindestens einen Antikörper nach einem der Ansprüche 1 bis 5.

10. Pharmazeutische Zusammensetzung umfassend mindestens einen Antikörper nach einem der Ansprüche 1 bis 5 und mindestens einen pharmazeutisch verträglichen Träger.

11. Impfstoff, umfassend mindestens einen Antikörper nach einem der Ansprüche 1 bis 5.

12. Verfahren zum *in vitro* Nachweis der A-Kette des Rizins in einer biologischen Probe, umfassend:
- das Inkontaktbringen der Probe mit mindestens einem Antikörper nach einem der Ansprüche 1 bis 5, und
- den Nachweis der Bindung des Antikörpers als Indikator für die Anwesenheit der genannten A-Kette des Rizins.

13. Immunkonjugat, umfassend einen Antikörper nach einem der Ansprüche 1 bis 5, welcher an einen therapeutischen Mittel gebunden ist.

## Claims

1. An anti-ricin antibody, **characterized in that** the variable region of the heavy chain has the amino acid sequence SEQ ID No. 1 and the variable region of the light chain has the amino acid sequence SEQ ID No. 2.

2. An anti-ricin scFv having the sequence SEQ ID No. 9.

3. The anti-ricin antibody according to claim 1 or 2, said antibody further being humanized or super-humanized.

4. The anti-ricin antibody according to claim 3, said antibody comprising at least one mutation in the variable region of the heavy chain chosen from none/Q (1), L/V (5), A/V (12), D/S (35), V/I (37), Y/R (50), W/Y (59), Q/K (76), T/S (78), F/Y (95), V/T (114) and L/V (116), the amino acid position being calculated from SEQ ID No. 1.

5. The anti-ricin antibody according to claim 3 or 4, said antibody comprising at least one mutation in the variable region of the light chain chosen from E/D (1), L/I (2), M/L (4), S/F (10), H/T (53), S/T (72) and V/T (85), the amino acid position being calculated from SEQ ID No. 2, or at least one mutation in the variable region of the light chain chosen from E/D (1), L/I (2), S/T (10), H/S (53), Q/E (55), S/T (72), E/D (81) and V/T (85), the amino acid position being calculated from SEQ ID No. 2.

6. A nucleic acid coding for an antibody according to any one of claims 1 to 5.

7. A vector comprising a nucleic acid according to claim 6.

8. A host cell comprising a vector according to claim 7.

9. A composition comprising at least one antibody according to any one of claims 1 to 5.

10. A pharmaceutical composition comprising at least one antibody according to any one of claims 1 to 5 and at least one pharmaceutically acceptable vehicle.

11. A vaccine comprising at least one antibody according to any one of claims 1 to 5.

12. A method for the *in-vitro* detection of the A-chain of the ricin in a biological sample, comprising:
- contacting the sample with at least one antibody according to any one of claims 1 to 5, and
- detecting the binding of said antibody as an indicator of the presence of said A-chain of the ricin.

13. An immunoconjugate comprising an antibody according to any one of claims 1 to 5 bound to a therapeutic agent.


### SEQUENCE LISTING

<110> Etat français, représenté par le Délégué Général de l'Armement THULLIER, Philippe
<120> ANTICORPS ANTI RICINE
<130> BCT080307 QT
<160> 10
<170> PatentIn version 3.3
<210> 1
   <211> 119
   <212> PRT
   <213> Macaca fascicularis
<400> 1
   Val Gln Leu Leu Glu Ser Gly Gly Gly Leu Ala Lys Pro Gly Gly Ser
   1               5                   10                  15
   Leu Arg Leu Ser Cys Ala Ala Ser Gly Phe Thr Phe Thr Asp Tyr Tyr
               20                  25                  30
   Met Asp Trp Val Arg Gln Ala Pro Gly Lys Gly Leu Glu Trp Val Ser
           35                  40                  45
   Arg Ile Ser Pro Gly Gly Asp Val Thr Trp Tyr Ala Asp Ser Val Lys
       50                  55                  60
   Gly Arg Phe Thr Ile Ser Arg Asp Asn Ala Gln Asn Thr Leu Tyr Leu
   65                  70                  75                  80
   Gln Met Asn Ser Leu Arg Ala Glu Asp Thr Ala Val Tyr Phe Cys Ala
                   85                  90                  95
   Arg Asp Asp Ile Val Val Ser Arg Ile Phe Asp Asp Trp Gly Gln Gly
               100                 105                 110
   Val Leu Leu Thr Val Ser Ser
           115
<210> 2
   <211> 107
   <212> PRT
   <213> Macaca fascicularis
<400> 2
   Glu Leu Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Arg Ser Tyr
               20                  25                  30
   Leu Ala Trp Tyr Gln Gln Lys Pro Gly Lys Ala Pro Lys Leu Leu Ile
           35                  40                  45
   Tyr Asp Ala Ala His Leu Gln Ser Gly Val Pro Ser Arg Phe Ser Gly
       50                  55                  60
   Ser Gly Ser Gly Thr Glu Phe Ser Leu Thr Ile Ser Ser Leu Gln Pro
   65                  70                  75                  80
   Glu Asp Phe Ala Val Tyr Tyr Cys Gln Gln Arg Asn Ser Tyr Pro Leu
                   85                  90                  95
   Thr Phe Gly Gly Gly Thr Lys Val Glu Ile Lys
               100                 105
<210> 3
   <211> 13
   <212> PRT
   <213> Macaca fascicularis
<400> 3
   Ala Arg Asp Asp Ile Val Val Ser Arg Ile Phe Asp Asp
   1               5                   10
<210> 4
   <211> 9
   <212> PRT
   <213> Macaca fascicularis
<400> 4
   Gln Gln Arg Asn Ser Tyr Pro Leu Thr
   1               5
<210> 5
   <211> 8
   <212> PRT
   <213> Macaca fascicularis
<400> 5
   Gly Phe Thr Phe Thr Asp Tyr Tyr
   1               5
<210> 6
   <211> 6
   <212> PRT
   <213> Macaca fascicularis
<400> 6
   Gln Ser Ile Arg Ser Tyr
   1 5
<210> 7
   <211> 8
   <212> PRT
   <213> Macaca fascicularis
<400> 7
   Ile Ser Pro Gly Gly Asp Val Thr
   1 5
<210> 8
   <211> 3
   <212> PRT
   <213> Macaca fascicularis
<400> 8
   Asp Ala Ala
   1
<210> 9
   <211> 245
   <212> PRT
   <213> Macaca fascicularis
<400> 9
   Gln Val Gln Leu Leu Glu Ser Gly Gly Gly Leu Ala Lys Pro Gly Gly
   1               5                   10                  15
   Ser Leu Arg Leu Ser Cys Ala Ala Ser Gly Phe Thr Phe Thr Asp Tyr
               20                  25                  30
   Tyr Met Asp Trp Val Arg Gln Ala Pro Gly Lys Gly Leu Glu Trp Val
           35                  40                  45
   Ser Arg Ile Ser Pro Gly Gly Asp Val Thr Trp Tyr Ala Asp Ser Val
       50                  55                  60
   Lys Gly Arg Phe Thr Ile Ser Arg Asp Asn Ala Gln Asn Thr Leu Tyr
   65                  70                  75                  80
   Leu Gln Met Asn Ser Leu Arg Ala Glu Asp Thr Ala Val Tyr Phe Cys
                   85                  90                  95
   Ala Arg Asp Asp Ile Val Val Ser Arg Ile Phe Asp Asp Trp Gly Gln
               100                 105                 110
   Gly Val Leu Leu Thr Val Ser Ser Ala Ser Thr Lys Gly Pro Lys Leu
           115                 120                 125
   Glu Glu Gly Glu Phe Ser Glu Ala Arg Val Glu Leu Gln Met Thr Gln
       130                 135                 140
   Ser Pro Ser Ser Leu Ser Ala Ser Val Gly Asp Arg Val Thr Ile Thr
   145                 150                 155                 160
   Cys Arg Ala Ser Gln Ser Ile Arg Ser Tyr Leu Ala Trp Tyr Gln Gln
                   165                 170                 175
   Lys Pro Gly Lys Ala Pro Lys Leu Leu Ile Tyr Asp Ala Ala His Leu
               180                 185                 190
   Gln Ser Gly Val Pro Ser Arg Phe Ser Gly Ser Gly Ser Gly Thr Glu
           195                 200                 205
   Phe Ser Leu Thr Ile Ser Ser Leu Gln Pro Glu Asp Phe Ala Val Tyr
       210                 215                 220
   Tyr Cys Gln Gln Arg Asn Ser Tyr Pro Leu Thr Phe Gly Gly Gly Thr
   225                 230                 235                 240
   Lys Val Glu Ile Lys
                   245
<210> 10
   <211> 18
   <212> PRT
   <213> artificial
<220>
   <223> lien peptidique
<400> 10
   Ala Ser Thr Lys Gly Pro Lys Leu Glu Glu Gly Glu Phe Ser Glu Ala
   1               5                   10                  15
   Arg Val